Europäisches Patentamt
European Patent Office
Office européen des brevets

(19) (11) Publication number: **0 022 626 A1**

(12) **EUROPEAN PATENT APPLICATION**

(21) Application number: **80302049.4**

(22) Date of filing: **18.06.80**

(51) Int. Cl.³: **C 07 C 79/35**, C 07 C 103/34, C 07 C 121/38, C 07 C 147/02, C 07 C 147/14, C 07 C 149/20, A 01 N 39/02

(30) Priority: **20.06.79 US 50284**
**29.05.80 US 154191**

(43) Date of publication of application: **21.01.81**
**Bulletin 81/3**

(84) Designated Contracting States: **AT BE CH DE FR GB IT LI LU NL SE**

(71) Applicant: **Rohm and Haas Company, Independence Mall West, Philadelphia, Pennsylvania 19105 (US)**

(72) Inventor: **Swithenbank, Colin, 1710 West Rock Road, Perkasie Pennsylvania 18944 (US)**
Inventor: **Fujimoto, Ted Tsutomu, 843 St. David's Avenue, Warminster Pennsylvania 18974 (US)**

(74) Representative: **Wood, Peter Worsley Spencer et al, Rohm and Haas Company Patent Department Chesterfield House Barter Street, London, WC1A 2TP (GB)**

(54) **Substituted nitrodiphenyl ethers, herbicidal compositions containing them and method of combating weeds.**

(57) Novel compounds are provided which exhibit activity as herbicides for combating weeds. The novel compounds have the formula:

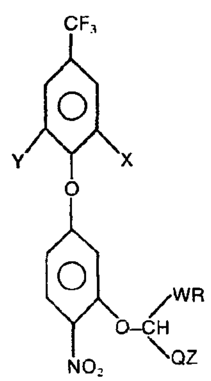

Formula I

wherein

X is hydrogen, halo, trifluoromethyl, $(C_1-C_4)$alkyl, cyano, or nitro;

Y is hydrogen, halo, or trifluoromethyl;

W is O or $S(O)_p$, wherein p is 0 to 2;

R is $(C_1-C_4)$alkyl, phenyl, halo-substituted phenyl, trifluoromethyl-substituted phenyl, or phenyl$(C_1-C_4)$alkyl;

Q is $(CH_2)_n$, wherein n is 0 to 4, or phenyl$(C_2-C_6)$alkenylene;

Z is $CO_2R^1$, $CO_2H$, $CO_2$, $CONH_2$, $CONHR^1$, $COSR^1$, $CON(R^1)_2$, CN, $CH_2OH$, $CH_2Cl$, $COR^1$, $CH_2OCH_3$, $CO_2(CR^2R^3)_mCO_2R^1$ or $OR^1$

wherein

$R^1$ is $(C_1-C_4)$alkyl, $(C_3-C_6)$alkenyl, cyano$(C_1-C_4)$alkyl, $(C_3-C_6)$alkynyl, $(C_2-C_4)$haloalkyl, $(C_1-C_4)$hydroxyalkyl, $(C_1$ to $C_4)$alkoxy$(C_1-C_4)$alkyl, $(C_1-C_4)$alkoxy$(C_1-C_4)$ alkoxy$(C_1$ to $C_4)$alkyl trifluoroethyl, $(C_2-C_4)$nitroalkyl, $(C_3-C_6)$cycloalkyl or phenyl$(C_1-C_4)$alkyl,

$R^2$ and $R^3$ are the same or different and are selected from hydrogen and methyl, the $-CR^2R^3-$groups being the same or different when m is > 1, and, when is $CO_2H$, the agronomically acceptable salts of compounds of Formula I.

EP 0 022 626 A1

TITLE MODIFIED
see front page

TITLE MODIFIED
see front page

0022626



Novel substituted nitrodiphenyl ethers, herbicidal compositions containing them and the use thereof for combating weeds

This invention concerns the provision of new nitrodiphenyl ethers, herbicidal compositions containing them, and methods of combating weeds.

Diphenyl ethers are known to be effective weed control agents. See for example U.S. Patent Nos. 3,928,416; 3,454,392; 3,798,276; 3,873,303; 4,001,005; 4,046,798; 4,063,929; 4,093,446 and 4,029,493 among the many patents issued. However, even now herbicidal effectiveness of a diphenyl ether cannot be predicted from its structure only, and often quite closely related compounds will have quite different weed control abilities. See, Advances in Agronomy, Vol. 24, pages 331, 332, 355, 356, 357 and 358, Herbicides, Chemical Degradation and Mode of Action, Kearney and Kaufman, Vol. 2, Dekker, Inc. pages 552-563 and 728-737 and Mode of Action of Herbicides, Ashton and Crafts and also U.S. Patent Nos. 3,454,392 and 3,776,961.

An ideal herbicide should give selective weed control, over the full growing season, with a single administration at low rates of application. It should be able to manage all common weeds by killing or controlling them as the seed, the germinating seed, the seedling, and the growing plant. At the same time, the herbicide should be substantially non-phytotoxic to the crops to which it is applied and should decompose or otherwise be dissipated under field conditions so as not to poison the soil permanently. Finally, they should be essentially nontoxic to animals. Known herbicides often fall short of one or more of these ideals and thus the search continues to discover new herbicides which are more selective or which complement the activities of known diphenyl ethers.

Nitrodiphenyl ethers within the scope of the present invention provide a useful choice in comparison with known nitrodiphenyl ether herbicides particularly as regards one or more of the following, viz good herbicidal activity, selectivity and reduced mammalian toxicity.

In accordance with the present invention, there is provided a new class of diphenyl ether herbicides having the following structural formula:

Formula I

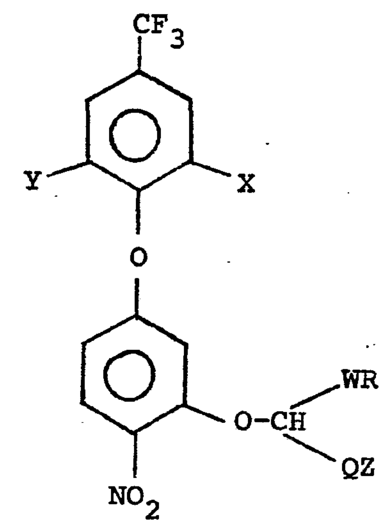

wherein X is hydrogen, halo, trifluoromethyl, $(C_1-C_4)$alkyl, cyano, or nitro;

Y is hydrogen, halo, or trifluoromethyl;

W is O or $S(O)_p$, wherein p is 0 to 2;

R is $(C_1-C_4)$alkyl, phenyl, halo substituted phenyl, or trifluoromethyl substituted phenyl, or phenyl-$(C_1-C_4)$alkyl;

Q is $(CH_2)_n$,wherein n is 0 to 4,or $(C_2-C_6)$alkenylene; and

Z is $CO_2R^1$, $CO_2H$, $CO_2^-$, $CONH_2$, $CONHR^1$, $COSR^1$, $CON(R^1)_2$, CN, $CH_2OH$, $CH_2Cl$, $COR^1$, $CH_2OCH_3$, $CO_2(CR^2R^3)_mCO_2R^1$ or $OR^1$ (particularly when n is

1, 2, 3 or 4 and $R^1$ is $(C_1-C_4)$alkyl), wherein

$R^1$ is $(C_1-C_4)$alkyl, $(C_3-C_6)$alkenyl, cyano$(C_1-C_4)$alkyl, $(C_3-C_6)$alkynyl, $(C_2-C_4)$haloalkyl, $(C_1-C_4)$-hydroxyalkyl, $(C_1-C_4)$alkoxy$(C_1-C_4)$alkyl, $(C_1-C_4)$-alkoxy$(C_1-C_4)$alkoxy$(C_1-C_4)$alkyl (ie R'O-R"O-R'"- where R" and R'" are $(C_1-C_4)$alkylene and R' is $(C_1-C_4)$alkyl), trifluoroethyl, $(C_2-C_4)$nitroalkyl, $(C_3-C_8)$cycloalkyl or phenyl$(C_1-C_4)$alkyl (particularly when Z is $CO_2R^1$);

$R^2$ and $R^3$ are the same or different and are selected from hydrogen and methyl, the $-CR^2R^3-$ groups being the same or different when m is >1 and, when Z is $CO_2H$, the agronomically acceptable salts of a compound of Formula I.

The term "halo" used throughout this specification means bromo, chloro, fluoro or iodo; preferred halo substituents are chloro or fluoro. The terms "alkyl", "alkoxy", "alkenyl" and "alkynyl" refer to all possible straight and branched chain isomeric forms so that, for example, "$(C_1-C_4)$alkyl" means each and every one of $CH_3$, $C_2H_5$, $C_3H_7$, $C_4H_9$ and all the isomers of the last two groups.

A narrower class of compounds has the formula:

Formula II

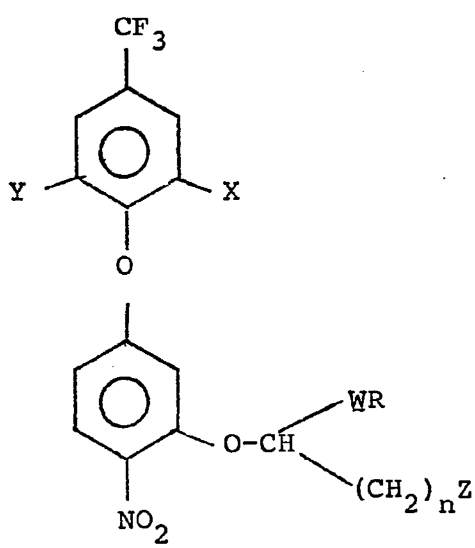

wherein X is hydrogen, halo; for example, bromo, chloro, fluoro, iodo, trifluoromethyl; alkyl, for example, lower alkyl of from 1 to 4 carbon atoms, preferably methyl or ethyl; cyano or nitro; more preferably, chloro;

Y is hydrogen, halo; for example, bromo, chloro, fluoro, iodo; or trifluoromethyl, preferably hydrogen;

W is O or $S(O)_p$, preferably O, S, or SO, and more preferably O;

R is lower alkyl of from 1 to 4 carbon atoms, phenyl, phenyl$(C_1-C_4)$alkyl such as benzyl, phenethyl, R preferably being methyl or ethyl;

n is from 0 to 4, preferably 0;

p is from 0 to 2, preferably 0 or 1;

Z is $CO_2R^1$, $CO_2H$, $CO_2^-$, $CONH_2$, $CONHR^1$, $COSR^1$, $CON(R^1)_2$, CN, $CH_2OH$, $CH_2Cl$, $COR^1$, or $CH_2OCH_3$, $R^1$ being $(C_1-C_4)$alkyl or $(C_2-C_4)$alkenyl and, when Z is COOH, the agronomically acceptable salts of Formula II.

Examples of salts of compounds of Formulae I and II (when Z is COOH) are the ammonia and amine salts, such as of triethylamine, ethanolamine, the alkali metal and alkaline earth metal salts such as those of Li, K, Na, Ca, Ba, Sr and Mg. It is preferred that the salts are storage-stable, eg at 18 - 25°C when present in closed containers.

A sub-class of compounds of Formula II and salts thereof (which are the subject of U.S. Patent Application Serial No. 50284 upon which the present application is partly based) are those in which X, Y, W, R, n and p are as defined for Formula II and Z is $CO_2R^1$, $CO_2H$, $CO_2^-$, $CONH_2$, $CONHR^1$, $CON(R^1)_2$, CN, $CH_2OH$, $CH_2Cl$ or $COCH_3$, $R^1$ being $(C_1-C_4)$alkyl. In this sub-class of compounds, it is preferred that X is cyano or chloro, particularly the latter, and Y is hydrogen.

A still narrower class of compounds has the formula:

Formula III

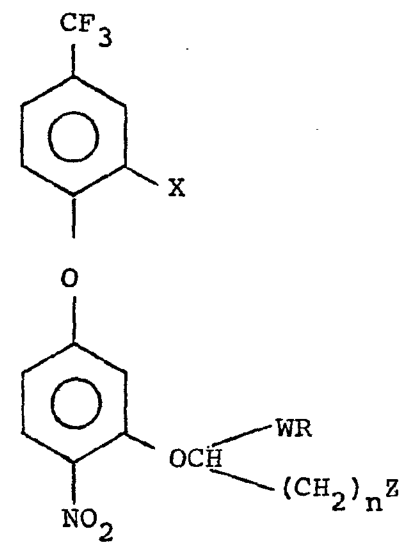

in which WR and Z have the meaning given above, and X is Cl or CN. Especially preferred compounds of Formula II are those in which X is Cl, WR is $OCH_3$ and Z is $CO_2R^1$ in which $R^1$ is $(C_1-C_4)$alkyl or $(C_3-C_4)$alkenyl.

Typical compounds of Formula III have the following values for WR, X and $(CH_2)_nZ$:

| WR | X | $(CH_2)_nZ$ | WR | X | $(CH_2)_nZ$ |
|---|---|---|---|---|---|
| $OCH_3$ | Cl | $CO_2CH_3$, | $OC_6H_5$ | Cl | $CO_2CH_3$, |
| $OCH_3$ | Cl | $CO_2Na$, | $OCH_2C_6H_5$ | Cl | $CO_2CH_3$, |
| $OC_2H_5$ | Cl | $CO_2CH_3$, | $OCH_3$ | Cl | $CO_2H$, |
| $SCH_3$ | Cl | $CO_2C_2H_5$, | $OCH_3$ | Cl | $CON(CH_3)_2$, |
| $SOCH_3$ | Cl | $CO_2C_2H_5$, | $OCH_3$ | Cl | $CH_2CO_2CH_3$, |
| $OCH_3$ | Cl | $CO_2C_2H_5$, | $OCH_3$ | CN | $CH_2CO_2CH_3$, |
| $OCH_3$ | Cl | $C_2H_4OH$, | $OCH_3$ | Cl | $CO_2CH_2CO_2CH_3$, |
| $OCH_3$ | Cl | $CO_2CH(CH_3)_2$, | $OCH_3$ | Cl | $CO_2CH(CH_3)CO_2CH_3$, |

| WR | X | $(CH_2)_nZ$ | WR | X | $(CH_2)_nZ$ |
|---|---|---|---|---|---|
| $OCH_3$ | Cl | $COCH_3$ | $OCH_3$ | Cl | $CO_2CH_2CH{=}CH_2$, |
| $SO_2CH_3$ | Cl | $CO_2CH_3$ | $OCH_3$ | Cl | CN, or |
| | | | $OCH_3$ | CN | $CO_2CH_3$, |

and similar radicals for Z in which n is O.

Among these, the following are particularly desirable:

| WR | X | $(CH_2)_nZ$ | WR | X | $(CH_2)_nZ$ |
|---|---|---|---|---|---|
| $OCH_3$ | Cl | $CO_2CH_3$, | $OCH_3$ | Cl | $CO_2CH_2CO_2CH_3$, |
| $OC_2H_5$ | Cl | $CO_2CH_3$, | $OCH_3$ | Cl | $CH_3$<br>$CO_2CHCO_2CH_3$, |
| $OCH_3$ | Cl | $CO_2C_2H_5$ | $OCH_3$ | Cl | $CO_2CH_2CH{=}CH_2$, |
| $OCH_3$ | Cl | $CO_2CH(CH_3)_2$ | $OCH_3$ | Cl | $CH_2CO_2CH_3$, or |
| $OC_6H_5$ | Cl | $CO_2CH_3$, | $OCH_3$ | Cl | CN |

A yet narrower class of compounds within the invention has the formula:

Formula IV

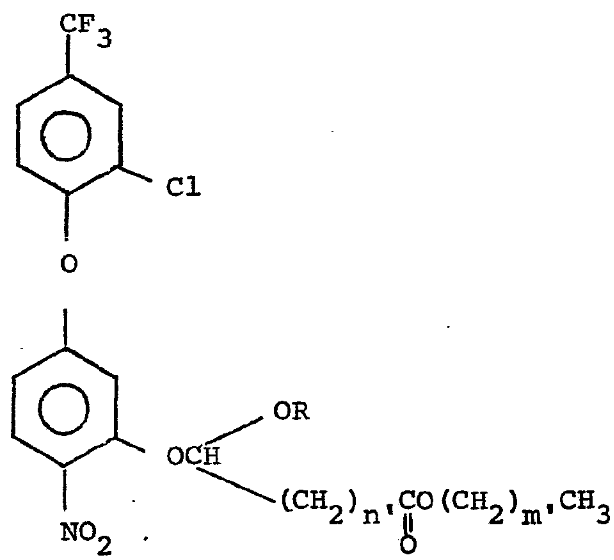

wherein R is $(C_1-C_4)$alkyl, for example methyl, ethyl, n-propyl, iso-propyl, n-butyl, sec-butyl, tert-butyl; and

n' and m' are 0 or 1. These compounds exhibit particularly good herbicidal activity, and have low toxicity to animals, particularly as to hematological toxicity.

The diphenyl ethers of Formula I are useful as preemergence and postemergence herbicides. Preemergence herbicides are ordinarily used to treat the soil by application either before seeding, during seeding, or after seeding and before the crop emerges. Postemergence herbicides are those which are applied after the plants have emerged and during their growth period. The compounds of this invention are especially active as postemergence herbicides.

Thus, the invention provides a method of combating weeds, usually in an agronomic crop area, which comprises applying to the growing weeds, eg weed seedlings, or to the growth medium prior to emergence of the weeds, one or more of the diphenyl ethers in an amount sufficient to combat the growth of the weeds.

When used in transplanted rice crops, the ethers can be applied either preemergence or postemergence to the weeds -- that is, they can be applied to the transplanted rice plants and their growth medium (eg paddy water) either before the weed plants have emerged or while they are in their early stages of growth. The ethers can be applied to the growth medium either before or after the rice has been transplanted to that medium.

The diphenyl ethers can be applied in any amount which will give the required control of weeds. A standard rate of application of the diphenyl ethers is from 0.0056 to 5.6 Kg/ha (0.005 to 5 pounds of diphenyl ether per acre). A preferred range is from 0.01 to 1.12 Kg/ha (0.01 to 1 pound per acre). The diphenyl ethers are particularly effective as post-emergent herbicides for weeds among the crops soybeans, peanuts, cotton, safflower, rape, sugarbeet and small grain crops such as wheat, barley oats and rice, at extremely low rates of application.

Under some conditions, the diphenyl ethers may be advantageously incorporated into the soil or other growth medium prior to planting a crop. This incorporation can be by any convenient means, including simple mixing with the soil, applying the diphenyl ether to the surface of the soil and then discing or dragging into the soil to the desired depth, or by employing a liquid carrier.

In their application as herbicides one or more of the compounds of Formula I will usually be used in conjunction with an agronomically acceptable carrier or diluent (and optionally a surfactant) so as to provide a herbicidal composition which, in general, will contain from 0.1 to 95% (more usually 1 to 95% and even more usually 5 to 95%) by weight of active herbicidal component comprising or consisting of one or more of the diphenyl ethers of Formula I. These herbicidal compositions may be in the form of ready-for-use dusts, solutions, emulsions, suspensions, granules or pellets; alternatively they may be in the form of concentrated solutions, wettable powders, water-dispersible granules, emulsifiable concentrates, flowable liquid suspensions, flowable emulsion concentrates, pastes or dust concentrates each for dilution with, as the case may be, additional liquid (usually water) or finely divided solids to give diluted solutions, suspensions, emulsions, or dusts which are then directly usable as herbicidal compositions.

By the term "agronomically acceptable carrier or diluent" as used above is meant a substance which can be utilized to dissolve, disperse, diffuse or otherwise dilute the active ingredient without impairing the effectiveness of

the active ingredient and which does no permanent damage to such loci as soil, equipment and agronomic crops.

It is usually desirable, particularly in postemergence applications, to include adjuvants, such as wetting agents, spreading agents, dispersing agents, stickers, adhesives, and the like, in accordance with agricultural practices. Examples of adjuvants which are commonly used in the art can be found in the John W. McCutcheon, Inc. publication "Detergents and Emulsifiers 1969 Annual".

Examples of solvents which are useful in the practice of this invention include alcohols, ketones, aromatic hydrocarbons, dimethylformamide, dioxane and dimethyl sulfoxide. Mixtures of these solvents can also be used. The concentration of the solution can vary from about 2% to about 98% by weight of diphenyl ether(s) with a preferred range being about 25% to about 75%.

For the preparation of emulsifiable concentrates, the diphenyl ether can be dissolved in organic solvents, such as benzene, toluene, xylene, methylated naphthalene, corn oil, pine oil, *o*-dichlorobenzene, isophorone, cyclohexanone, methyl oleate, and the like, or in mixtures of these solvents, together with an emulsifying agent which permits dispersion in water. Suitable emulsifiers include ethylene oxide derivatives of alkylphenols or long-chain alcohols, mercaptans, carboxylic acids, and reactive amines and partially esterified polyhydric alcohols. Solvent-soluble sulfates or sulfonates, such as the alkaline earth salts or amine salts of alkylbenzenesulfonates and the fatty alcohol sodium sulfates, having surface-active properties can be used as emulsifiers either alone or in conjunction with an ethylene oxide reaction product. Flowable emulsion concentrates are formulated similarly to the emulsifiable concentrates and include, in addition to

the above components, water and a stabilizing agent such as a water-soluble cellulose derivative or a water-soluble salt of a polyacrylic acid. The concentration of the active ingredient in emulsifiable concentrates is usually about 10% to 60% by weight and in flowable emulsion concentrates, and can be as high as about 75% by weight.

Wettable powders suitable for spraying, can be prepared by admixing the compound with a finely divided solid, such as clays, inorganic silicates and carbonates, and silicas and then incorporating wetting agents, sticking agents, and/or dispersing agents. The concentration of active ingredients in such formulations is usually in the range of about 20% to about 98%, preferably about 40% to about 75% by weight. A dispersing agent can constitute about 0.5% to about 3% by weight of the composition, and a wetting agent can constitute from about 0.1% to about 5% by weight of the composition.

Dusts can be prepared by mixing the compounds of the invention with finely divided inert solids which may be organic or inorganic in nature. Materials useful for this purpose include, for example, botanical flours, silicas, silicates, carbonates and clays. One convenient method of preparing a dust is to dilute a wettable powder with a finely divided carrier. Dust concentrates containing about 20% to about 80% by weight of the active ingredient are commonly made and are subsequently diluted to about 1% to about 10% by weight use concentration.

Granular formulations can be prepared by impregnating a solid such as granular fuller's earth, vermiculite, ground corn cobs, seed hulls, including bran or other grain-hulls, or similar material. A solution of one or more of the diphenyl ethers in a volatile organic solvent can be sprayed or mixed with the granular solid and the solvent then removed by evaporation. The granular material can have any suitable size, with a preferable size range of 16 to 60 mesh. The diphenyl ether will usually comprise from about 2 to about 15% by weight of the granular formulation.

The diphenyl ethers of the invention can also be mixed with fertilizers of fertilizing materials before their application. In one type of solid fertilizing composition in which the diphenyl ethers can be used, particles of a fertilizer or fertilizing ingredients, such as ammonium sulfate, ammonium nitrate, or ammonium phosphate, can be coated with one or more of the ethers. The solid diphenyl ethers and solid fertilizing material can also be admixed in mixing or blending equipment, or they can be incorporated with fertilizing in granular formulations. Any relative proportion of diphenyl ether and fertilizer can be used which is suitable for the crops and weeds to be treated. The diphenyl ether will commonly be used from about 5% to about 25% by weight of the fertilizing composition. These compositions provide fertilizing materials which promote the rapid growth of desired plants, and at the same time control the growth of undesired plants.

The diphenyl ethers of the invention can be applied as herbicidal sprays by methods commonly employed such as conventional high-gallonage hydraulic sprays, low-gallonage sprays, airblast spray, aerial sprays and dusts. For low volume applications a solution of the compound is usually used. The dilution and rate of application will usually depend upon such factors as the type of equipment employed, the method of application, the area to be treated and the type and stage of development of the weeds.

For some applications, it may be desirable to use the diphenyl ethers of Formula I in conjunction (usually in admixture) with other herbicides such as those listed in The

Pesticide Manual, the British Crop Protection Council; Sixth Edition, 1979 and those listed in Applicant's Australian Published Patent Specification No. 506,509 (Application No. 28372/77).

The following examples are given by way of illustration. Percentages are on a weight basis unless otherwise indicated.

Example 1

Preparation of Methyl 2-methoxy-2-{2-nitro-5-(2-chloro-4-trifluoromethylphenoxy)phenoxy}acetate

The potassium salt of 2-chloro-4-trifluoromethyl-3'-hydroxy-4'-nitrodiphenylether (9 g, vide U.S. Patent No. 4,093,446 for preparation) in dimethyl sulfoxide was reacted with methyl α-chloro-α-methoxyacetate {4.3 g, vide Gross and Freiberg, Chem. Ber. 99, 3260, (1966)} at room temperature. The color quickly changed from orange red to pale yellow and the reaction mixture was taken up in ether and washed several times with aqueous 0.05% sodium hydroxide and, with water, dried, filtered through activated silica gel and the solvent removed to give the desired ester as an oil (5.4 g), which upon standing crystalized to give a product which melted at 59-60°C. Found C, 46.79; H, 2.87; N, 3.73; Cl, 8.30: F, 13.01. $C_{17}H_{13}ClF_3NO_7$ requires C, 46.86; H, 3.01; N, 3.21; Cl, 8.14; F, 13.08.

This compound has the formula:

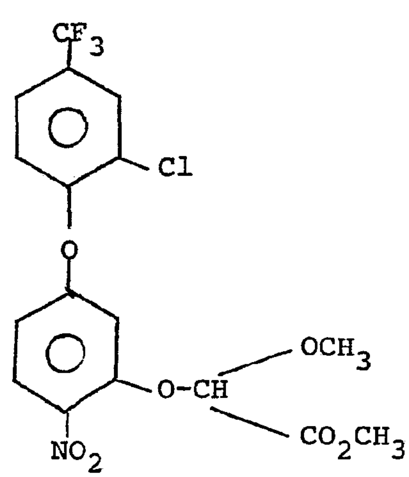

Alternative nomenclature is methyl α-{-methoxy-α-{(2-nitro-5-(2-chloro-4-trifluoromethylphenoxy)phenoxy}-acetate or 2-chloro-4-trifluoromethyl-3'-(1-methoxy-1-methoxycarbonyl-methoxy)-4'-nitrodiphenyl ether.

Example 2

Preparation of 2-chloro-4-trifluoromethyl-3'-(1-methoxy-1-carboxymethoxy)-4'-nitrodiphenyl ether

2-Chloro-4-trifluoromethyl-3'-(1-methoxy-1-methoxycarbonyl-methoxy)-4'-nitrodiphenyl ether (10 g) was dissolved in methanol/water (100 ml/9 ml) and the solution cooled to 10°C in an ice bath. A solution of aqueous 50% NaOH (3.5 g) in methanol (25 ml) was added and the mixture stirred for 5 minutes. The reaction mixture was poured on to ice, extracted with ether and the extract discarded. The aqueous phase was neutralized with aqueous 5% $H_2SO_4$ (88 ml), extracted with methylene chloride, and the extract dried and stripped to give 2-chloro-4-trifluoromethyl-3'-(1-methoxy-1-carboxymethoxy)-4'-nitrodiphenyl ether (9.2 g, 95%). $^1H$ n.m.r. of 3' side-chain δ($CDCl_3$) 5.6 (s, 1H), 3.6 (s, 3H).

The free acid prepared as above disproportionates slowly at room temperature (18-25°C) and its sodium salt disproportionates within about 24 hours. The sodium salt was prepared by the reaction in solution of the free acid and sodium hydroxide.

Example 3

Preparation of 2-chloro-4-trifluoromethyl-3'-(1-methoxy-1-ethoxycarbonylmethoxy)-4'-nitrodiphenyl ether

2-Chloro-4-trifluoromethyl-3'-(1-methoxy-1-methoxycarbonyl-methoxy)-4'-nitrodiphenyl ether (5 g) was dissolved in ethanol (50 ml). Separately, NaH (.1 g) was dissolved in ethanol (5 ml). The two solutions were combined and heated at 63°C for three hours. The reaction mixture was diluted

with 50/50 by volume ether/hexane and the organic phase washed with water, dried, filtered through neutral alumina and stripped to give 2-chloro-4-trifluoromethyl-3'-(1-methoxy-1-ethoxycarbonylmethoxy)-4'-nitrodiphenyl ether (1.1 g, 21%). $^1$H n.m.r. of 3' side-chain $\delta$ ($CDCl_3$) 5.6 (s, 1H), 4.3 (quart., 2H), 3.6 (s, 3H), 1.3 (t, 3H).

Example 4

Preparation of 2-chloro-4-trifluoromethyl-3'-(1-methoxy-1-isopropoxycarbonylmethoxy)-4'-nitrodiphenyl ether

2-Chloro-4-trifluoromethyl-3'-(1-methoxy-1-methoxycarbonylmethoxy)-4'-nitrodiphenyl ether (5 g) was dissolved in isopropanol (10 ml) and heated to 65°C with stirring. NaH (.5 g) was dissolved in isopropanol and added in aliquots to the above solution until the reaction was complete. The reaction mixture was diluted with ether and the ether phase washed with water, dried, filtered through paper and stripped to give the desired product (.8 g, 15%). $^1$H n.m.r. of 3' side-chain $\delta$ ($CDCl_3$) 5.4 (s, 1H), 5.0 (sept., 1H), 3.5 (s, 3H), 1.1 (d, 6H).

Example 5

Preparation of 2-chloro-4-trifluoromethyl-3'-(1-methoxy-1-allyloxycarbonylmethoxy)-4'-nitrodiphenyl ether

2-Chloro-4-trifluoromethyl-3'-(1-methoxy-1-methoxycarbonylmethoxy)-4'-nitrodiphenyl ether (5 g) was dissolved in allyl alcohol (50 ml) and combined with NaH (.3 g), predissolved in allyl alcohol (10 ml). The reaction mixture was stirred at room temperature overnight, then diluted with ether/hexane (50/50 by volume), the organic phase washed with water, dried, filtered through activated alumina and stripped to give 2-chloro-4-trifluoromethyl-3'-(1-methoxy-1-allyloxycarbonylmethoxy)-4'-nitrodiphenyl ether (1.9 g, 36%). $^1$H n.m.r. of 3' side-chain $\delta$ ($CDCl_3$) 5.5 (s, 1H), 5.1-6.1 (m, 3H), 4.7 (d, 2H), 3.7 (s, 3H).

Example 6

Preparation of 2-chloro-4-trifluoromethyl-3'-(1-ethoxy-1-ethoxycarbonylmethoxy)-4'-nitrodiphenyl ether

2-Chloro-4-trifluoromethyl-3'-hydroxy-4'-nitrodiphenyl ether (1 equivalent) was dissolved in methylethyl ketone and an excess over the equivalent amount of anhydrous potassium carbonate was added. The reaction mixture was heated under reflux with stirring for 15-30 minutes to form the potassium salt, then cooled to room temperature. Ethyl $\alpha$-chloro-$\alpha$-ethoxyacetate (1 equivalent, prepared as in Ber. 44:3213, Compt. Rend. 152:960) was added and the mixture stirred for 15-30 minutes. The reaction mixture was diluted with ether, or ether/hexane (50/50 by volume), and the extract washed with water, dried, filtered through activated alumina and stripped to give 2-chloro-4-trifluoromethyl-3'-(1-ethoxy-1-ethoxycarbonylmethoxy)-4'-nitrodiphenyl ether. $^1$H n.m.r. of 3' side-chain $\delta$ ($CDCl_3$) 5.6 (s, 1H), 4.2 (quart., 2H), 3.8 (quart. 2H), 1.2 (2t, 6H).

Example 7

Preparation of 2-chloro-4-trifluoromethyl-3'-(1-benzyloxy-1-methoxycarbonylmethoxy-4'-nitrodiphenyl ether

2-Chloro-4-trifluoromethyl-3'-(1-benzyloxy-1-benzyloxycarbonylmethoxy)-4'-nitrodiphenyl ether (2.3 g) was dissolved in methanol (25 ml) and combined with a solution of NaH (.05 g) in methanol. The reaction mixture was stirred at room temperature for three hours, then diluted with ether, the ether layer washed with water, dried, filtered through alumina and stripped giving 2-chloro-4-trifluoromethyl-3'-(1-benzyloxy-1-methoxycarbonylmethoxy)-4'-nitrodiphenyl ether (.5 g, 25%). $^1$H n.m.r. $\delta$ ($CDCl_3$) 6.5-8.0 (m, 11H), 5.6 (s, 1H), 4.8 (s, 2H), 3.7 (s, 3H).

Example 8

Preparation of 2-chloro-4-trifluoromethyl-3'-(1-benzyloxy-1-benzyloxycarbonylmethoxy)-4'-nitrodiphenyl ether

Methyl α,α-dimethoxy acetate (10 g), benzyl alcohol (24.3 g) and p-toluene sulfonic acid (.1 g) were combined and heated to 130°C, with stirring, for approximately 12 hours. Since several products were present, the reaction sample was purified by preparative high performance liquid chromatography to give 5.1 gms of the pure product. Benzyl α-benzyloxy-α-methoxy acetate was then chlorinated (Ber. 44: 3213, Mylo) to give benzyl benzyloxychloroacetate. This reagent was used to alkylate the potassium salt 2-chloro-4-trifluoro-methyl-3'-hydroxy-4'-nitrodiphenyl ether using the procedure given in Example 6 to yield 2-chloro-4-trifluoromethyl-3'-(1-benzyloxy-1-benzyloxycarbonylmethoxy)-'4-nitrodiphenyl ether. $^1H$ n.m.r. δ ($CDCl_3$) 6.3-7.9 (m, 16H),5.6 (s, 1H), 5.1 (s, 2H), 4.7 (s, 2H).

Example 9

Preparation of 2-chloro-4-trifluoromethyl-3'-(1-methylthio-1-ethoxycarbonylmethoxy)-4'-nitrodiphenyl ether

Methyl α-chloro-α-methylthioacetate was synthesized following the published procedure of Weygard and Bestman, Ber. 89:1912 (1956). This material was then used to alkylate the potassium salt of 2-chloro-4-trifluoromethyl-3'-hydroxy-4'-nitrodiphenyl ether following the procedure given in Example 6 to give 2-chloro-4-trifluoromethyl-3'-(1-methylthio-1-ethoxy-carbonylmethoxy)-4'-nitrodiphenyl ether. $^1H$ n.m.r. of 3' side-chain δ ($CDCl_3$) 5.5 (s, 1H), 4.2 (quart., 2H), 2.2 (s, 3H), 1.3 (t, 3H).

Example 10

Preparation of 2-chloro-4-trifluoromethyl-3'-(1-methylsulfinyl-1-ethoxycarbonylmethoxy)-4'-nitrodiphenyl ether

2-Chloro-4-trifluoromethyl-3'-(1-methylthio-1-ethoxycarbonylmethoxy)-4'-nitrodiphenyl ether (1 g) was dissolved in 30 ml methylene chloride and cooled with stirring in an ice bath to 1°C. Separately, m-chloroperoxybenzoic acid, 85% pure (.35 g) was dissolved in 2 ml of methylene chloride

and also cooled to 1°C. The peroxide solution was then added dropwise to the diphenyl ether solution. After the addition was complete, the reaction was warmed to room temperature. The methylene chloride solution was washed with aqueous 5% $Na_2SO_3$ and then with water, dried, filtered through alumina and stripped to yield 2-chloro-4-trifluoromethyl-3'-(1-methylsulfinyl-1-ethoxycarbonylmethoxy)-4'-nitrodiphenyl ether (.1 g). $^1H$ n.m.r. of 3' side-chain $\delta$ ($CDCl_3$) 5.3 (s, 1H), 4.3 (quart., 2H), 2.7 (s, 3H), 1.2 (t, 3H).

Example 11

Preparation of 2-chloro-4-trifluoromethyl-3'-(1-methylsulfonyl-1-ethoxycarbonylmethoxy)-4-nitrodiphenyl ether

2-Chloro-4-trifluoromethyl-3'-(1-methylthio-1-ethoxycarbonylmethoxy)-4'-nitrodiphenyl ether (1.4 g) was dissolved in methylene chloride (50 ml) and heated to reflux. Separately, m-chloroperoxybenzoic acid (0.7 g) was dissolved in methylene chloride (2 ml) and was then added in aliquots to the above solution until the reaction was complete. The reaction mixture was washed with water, dried, filtered through paper several times and stripped to give 2-chloro-4-trifluoromethyl-3'-(1-methylsulfonyl-1-ethoxycarbonylmethoxy)-4'-nitrodiphenyl ether (1.3 g). $^1H$ n.m.r. of 3' side-chain $\delta$ ($CDCl_3$) 5.4 (s, 1H), 4.3 (quart., 2H), 3.2 (s, 3H), 1.3 (t, 3H).

Example 12

Preparation of 2-chloro-4-trifluoromethyl-3'-(1-methoxy-1-cyanomethoxy)-4'-nitrodiphenyl ether

$\alpha$-Methoxyacetonitrile (25 g) was dissolved in carbon tetrachloride (80 ml) and to this solution was added N-bromosuccinimide (21.4 g) and azobisisobutyronitrile (3.7 g). The reaction mixture was heated at reflux for a total of 10 hours giving at least 50% conversion to the product. The carbon tetrachloride was stripped and the residue distilled. The product distilled at 29-33°C and 1 mm pressure giving 16.4 gms of α-bromo-α-methoxy acetonitrile. This reagent was used to alkylate the potassium salt 2-chloro-4-trifluoromethyl-3'-hydroxy-4'-nitrodiphenyl ether following the procedure given in Example 6 to give 2-chloro-4-trifluoromethyl-3'-(1-methoxy-1-cyanomethoxy)-4'-nitrodiphenyl ether. $^1$H n.m.r. of 3' side-chain $\delta$ ($CDCl_3$) 5.9 (s, 1H), 3.7 (s, 3H).

Example 13

Preparation of 2-chloro-4-trifluoromethyl-3'-(1-phenoxy-1-cyanomethoxy)-4'-nitrodiphenyl ether

α-Phenoxyacetonitrile (3.4 g), N-bromosuccinimide (5.6 g), azo bisisobutyronitrile (.56 g), and carbon tetrachloride (30 ml) were combined and heated with stirring to reflux. A General Electric sunlamp was directed at the reactor and after 20 hours the reaction was complete. The reaction mixture was dissolved in hexane, washed with water, dried with anhydrous $NaSO_4$, filtered through silica gel and stripped to give α-phenoxy-α-bromo acetonitrile (1.1 g). This material was used to alkylate the potassium salt of 2-chloro-4-trifluoromethyl-3'-hydroxy-4'-nitrodiphenyl ether by the procedure described in Example 6 to give 2-chloro-4-trifluoromethyl-3'-(1-phenoxy-1-cyanomethoxy)-4'-nitrodiphenyl ether. $^1$H n.m.r. of 3' side-chain $\delta$ ($CDCl_3$) 6.7-8.1 (m, 11H), 6.5 (s, 1H).

Example 14

Preparation of 2-chloro-4-trifluoromethyl-3'-(1-methoxy-1-dimethylaminocarbonylmethoxy)-4'-nitrodiphenyl ether

Dimethylamine gas was bubbled through methyl α,α-dimethoxyacetate (27 g ) containing sodium methoxide (1 g) for 3.5 hours. The reaction mixture was then filtered through paper and stripped giving N,N-dimethyl-α,α-dimethoxyacetamide (24.9 g). This was then combined with acetyl chloride (13.3 g) and heated to 60°C with stirring for

approximately 4 hours. The reaction was then filtered through paper and stripped giving N,N-dimethyl-α-chloro-α-methoxyacetamide (24.4 g). This material was used to alkylate the potassium salt of 2-chloro-4-trifluoromethyl-3'-hydroxy-4'-nitrodiphenyl ether following the procedure given in Example 6 to give 2-chloro-4-trifluoromethyl-3'-(1-methoxy-1-dimethylaminocarbonylmethoxy)-4'-nitrodiphenyl ether. $^1$H n.m.r. of 3' side-chain $\delta$ ($CDCl_3$) 5.5 (s, 1H), 3.5 (s, 3H), 3.1 (s, 3H), 2.9 (s, 3H).

<u>Example 15</u>

<u>Preparation of 2-chloro-4-trifluoromethyl-3'-(1-methoxy-1-acetylmethoxy)-4'-nitrodiphenyl ether</u>

Methyl glyoxal dimethyl acetal (10 g), acetyl chloride (6.7 g), and benzyltriethylammonium chloride (100 mg) were heated under reflux for approximately 4 hours. The reaction mixture was then stripped and distilled, the 1-chloro-1-methoxy-2-oxopropane (7.6 g) distilling over at 70°C and 65 mm Hg. This material was then used to alkylate the potassium salt of 2-chloro-4-trifluoromethyl-3'-hydroxy-4'-nitrodiphenyl ether following the procedure of Example 6 yielding 2-chloro-4-trifluoromethyl-3'-(1-methoxy-1-acetylmethoxy)-4'-nitrodiphenyl ether. $^1$H n.m.r. of 3' side-chain $\delta$ ($CDCl_3$) 5.3 (s, 1H), 3.5 (s, 3H), 2.4 (s, 3H).

<u>Example 16</u>

<u>Preparation of 2-chloro-4-trifluoromethyl-3'-(1,2-dimethoxyethoxy)-4'-nitrodiphenyl ether</u>

Methoxy acetaldehyde dimethyl acetal (10 g), acetyl chloride (6.3 g) and benzyltriethylammonium chloride (10 mg) were combined and heated to reflux for 1 hour. The reaction mixture was stripped and then distilled. 2-chloro-1,1-dimethoxyethane (3.8 g) distilled at 62°C and 80 mm Hg. This material was used to alkylate the potassium salt of 2-chloro-4-trifluoromethyl-3'-hydroxy-4'-nitrodiphenyl ether by the procedure described in Example 6 to give 2-chloro-4-trifluoromethyl-3'-(1,2-dimethoxyethoxy)-4'-nitrodiphenyl ether. $^1$H n.m.r. of 3' side-chain $\delta$ ($CDCl_3$) 5.4 (t, 1H), 3.7 (d, 2H), 3.4 (s, 3H), 3.3 (s, 3H).

Example 17

Preparation of 2-chloro-4-trifluoromethyl-3'-(1-methoxy-3-methoxycarbonylpropoxy)-4'-nitrodiphenyl ether

Methyl 4-oxo-*n*-butyrate was synthesized from mono-methylsuccinate (49.8 g) by following the published procedure {Syn 767 (1976)}. The methyl 4-oxo-*n*-butyrate was then dissolved in methanol with stirring for approximately 1 hour forming methyl 4,4-dimethoxy-*n*-butyrate. The methanol was then stripped from the reaction and the remainder was distilled. Methyl 4,4-dimethoxy-*n*-butyrate codistilled with an impurity, dimethylsuccinate, at 112°C and 70 mm Hg. (15.7 g).

Methyl 4,4-dimethoxy-*n*-butyrate (15.7 g), acetyl chloride (10 g) and copper bronze (100 mg) was combined and heated under reflux for 2 hours. The reaction mixture was filtered through paper and stripped to yield methyl 4-chloro-4-methoxy-*n*-butyrate plus impurities (13.2 g). This material was used to alkylate the potassium 2-chloro-4-trifluoromethyl-3'-hydroxy-4'-nitrodiphenyl ether by the procedure of Example 6 to give 2-chloro-4-trifluoromethyl-3'-(1-methoxy-3-methoxycarbonylpropoxy)-4'-nitrodiphenyl ether. $^1$H n.m.r. of 3' side-chain $\delta$ ($CDCl_3$) 5.4 (t, 1H), 3.7 (s, 3H), 3.4 (s, 3H), 2.1-2.7 (m, 4H).

Example 18

Preparation of 2-chloro-4-trifluoromethyl-3'-{1-methoxy-1-(methoxycarbonylmethoxycarbonyl)methoxy}-4'-nitrodiphenyl ether

Methyl $\alpha,\alpha$-dimethoxyacetate (10 g), powdered NaOH (3 g) and methanol (50 ml) were combined and heated to reflux for 20 hours. The methanol was stripped giving sodium $\alpha,\alpha$-di-

methoxyacetate.

Sodium α,α-dimethoxyacetate (7.7 g), methyl α-bromoacetate (8.3 g), methyl ethyl ketone (25 ml), benzyl triethylamonium bromide (200 mg) and sodium iodide (.5 g) were combined and heated to reflux for 20 hours. The reaction was diluted with ether, filtered through paper and stripped to give methoxycarbonylmethyl α,α-dimethoxy acetate (8.9 g) and this compound was converted to a methoxycarbonylmethyl α-chloro-α-methoxy acetate following Mylo's procedure (loc cit.: Ber 44:3213). This material was then used to alkylate the potassium salt of 2-chloro-4-trifluoromethyl-3'-hydroxy-4'-nitrodiphenyl ether following the procedure given in Example 6 to give 2-chloro-4-trifluoromethyl-3'-{1-methoxy-1-(methoxycarbonylmethoxycarbonyl)methoxy}-4'-nitrodiphenyl ether. $^1H$ n.m.r. of 3' side-chain $\delta$ ($CDCl_3$) 5.6 (s, 1H), 4.6 (s, 2H), 3.6 (s, 3H), 3.5 (s, 3H).

<u>Example 19</u>

<u>Preparation of 2-chloro-4-trifluoromethyl-3'-{1-methoxy-1-(1-methoxycarbonylethoxycarbonyl)methoxy}-4'-nitrodiphenyl ether</u>

Sodium α,α-dimethoxyacetate (9.6 g), methyl α-bromopropionate (11.3 g), methyl ethyl ketone (60 ml), benzyltrimethylammonium bromide (200 mg) and sodium iodide (.5 g) were combined and heated at reflux for 2 days. The reaction mixture was diluted with ether, filtered through paper and stripped to give (1-methoxycarbonylethyl)dimethoxy acetate (11.2 g).

(1-Methoxycarbonylethyl)chloromethoxy acetate was synthesized from (1-methoxycarbonylethyl)dimethoxy acetate following the procedure Ber. <u>44</u>:3213 Mylo. This material was used to alkylate 2-chloro-4-trifluoromethyl-3'-hydroxy-4'-nitrodiphenyl ether by the procedure given in Example 6 to give 2-chloro-4-trifluoromethyl-3'-{1-methoxy-1-(1-methoxycarbonylethoxycarbonyl)methoxy}-4'-nitrodiphenyl ether. $^{1}H$ n.m.r. of 3' side-chain $\delta$ ($CDCl_3$) 5.6 (s, 1H), 5.1 (quart., 1H), 3.7 (s, 3H), 3.6 (s, 3H), 1.5 (d, 3H).

The -OCH(WR)QZ group vide Formula I of the compounds of the foregoing examples are as follows:

Example

1. $OCH(OMe)CO_2Me$
2. $OCH(OMe)CO_2H$ (plus Na salt)
3. $OCH(OMe)CO_2Et$
4. $OCH(OMe)CO_2$*i*-Pr
5. $OCH(OMe)CO_2CH_2CH{=}CH$
6. $OCH(OEt)CO_2Et$
7. $OCH(OCH_2Ph)CO_2Me$
8. $OCH(OCH_2Ph)CO_2CH_2Ph$
9. $OCH(SMe)CO_2Et$
10. $OCH(SOMe)CO_2Et$
11. $OCH(SO_2Me)CO_2Et$
12. OCH(OMe)CN
13. OCH(OPh)CN
14. $OCH(OMe)CONMe_2$
15. OCH(OMe)COMe
16. $OCH(OMe)CH_2OMe$
17. $OCH(OMe)CH_2CH_2CO_2Me$
18. $OCH(OMe)CO_2CH_2CO_2Me$
19. $OCH(OMe)CO_2CH(Me)CO_2Me$

In the above list, as is conventional, Me = methyl, Et = ethyl, *i*-Pr = isopropyl and Ph = phenyl.

Table I below gives physical data for the compounds of Examples 1 - 19, the extreme left hand column giving the respective Example number.

TABLE I

Diphenylethers - Physical Data

| | m.p. | Formula | C Found | C (req'd) | H Found | H (req'd) | N Found | N (req'd) | Cl Found | Cl (req'd) |
|---|---|---|---|---|---|---|---|---|---|---|
| 1 | 59-60°C | $C_{17}H_{13}ClF_3NO_7$ | 46.79 | (46.86) | 2.87 | ( 3.01) | 3.73 | (3.21) | 8.30 | ( 8.14) |
| 2 | oil | $C_{16}H_{11}ClF_3NO_7$ | 42.24 | (45.46) | 2.67 | ( 2.62) | 3.29 | ( 3.31) | 12.52 | (13.72) |
| 3 | oil | $C_{18}H_{15}ClF_3NO_7$ | 48.67 | (47.96) | 3.43 | ( 3.35) | 3.07 | ( 3.11) | 7.79 | ( 7.87) |
| 4 | oil | $C_{19}H_{17}ClF_3NO_7$ | 49.92 | (49.10) | 3.69 | ( 3.69) | 2.75 | ( 3.01) | 7.39 | ( 7.63) |
| 5 | oil | $C_{19}H_{15}ClF_3NO_7$ | 50.33 | (49.31) | 3.41 | ( 3.27) | 2.92 | ( 3.02) | 7.57 | ( 7.66) |
| 6 | oil | $C_{19}H_{17}ClF_3NO_7$ | 47.69 | (49.21) | 3.94 | ( 3.69) | 3.20 | ( 3.02) | 9.66 | ( 7.64) |
| 7 | oil | | Obtained as a 50% mixture with benzyl alcohol. Structure proof by n.m.r. See Example 7. | | | | | | | |
| 8 | oil | $C_{29}H_{21}ClF_3NO_7$ | 58.70 | (59.14) | 3.63 | ( 3.59) | 2.74 | ( 2.38) | 6.46 | ( 6.02) |
| 9 | oil | $C_{18}H_{15}ClF_3NO_6S$ | 44.31 | (46.41) | 3.13 | ( 3.25) | 2.91 | ( 3.01) | 8.16 | ( 7.61) |
| 10 | oil | $C_{18}H_{15}ClF_3NO_7S$ | 44.07 | (44.87) | 3.23 | ( 3.14) | 2.74 | ( 2.91) | - | |
| 11 | oil | $C_{18}H_{15}ClF_3NO_8S$ | 43.45 | (43.43) | 2.98 | ( 3.04) | 2.60 | ( 2.81) | 9.59 | ( 7.12) |
| 12 | oil | $C_{16}H_{10}ClF_3N_2O_5$ | 48.50 | (47.72) | 2.93 | ( 2.50) | 7.22 | ( 6.96) | 8.36 | ( 8.80) |
| 13 | oil | $C_{21}H_{12}ClF_3N_2O_5$ | 53.94 | (54.27) | 2.68 | ( 2.60) | 6.33 | ( 6.03) | 7.70 | ( 7.63) |
| 14 | oil | $C_{18}H_{16}ClF_3N_2O_6$ | 48.15 | (48.17) | 3.57 | ( 3.59) | 6.62 | ( 6.24) | 7.78 | ( 7.90) |
| 15 | oil | $C_{17}H_{13}ClF_3NO_6$ | 48.55 | (48.53) | 3.16 | ( 3.11) | 3.62 | ( 3.33) | 8.38 | ( 8.43) |
| 16 | oil | $C_{17}H_{15}ClF_3NO_6$ | 48.23 | (48.30) | 3.58 | ( 3.58) | 3.59 | ( 3.31) | 8.18 | ( 8.39) |
| 17 | oil | $C_{19}H_{17}ClF_3NO_7$ | 48.91 | (49.21) | 3.97 | ( 3.69) | 2.51 | ( 3.02) | 7.75 | ( 7.64) |
| 18 | oil | $C_{19}H_{15}ClF_3NO_9$ | 45.99 | (46.12) | 3.26 | ( 3.06) | 2.92 | ( 2.83) | 7.47 | ( 7.17) |
| 19 | oil | $C_{20}H_{17}ClF_3NO_9$ | 47.34 | (47.31) | 3.40 | ( 3.37) | 3.59 | ( 2.76) | 7.41 | ( 6.98) |

Typical examples of other compounds of the invention embraced by Formula I include:

Methyl 2-ethoxy-2-[2-nitro-5-(2-chloro-4-trifluoromethylphenoxy)phenoxy]acetate,

Methyl 2-n-butoxy-2-[2-nitro-5-(2-fluoro-4-trifluoromethyl-6-chlorophenoxy)-phenoxy]acetate,

Ethyl 2-methylthio-2-[2-nitro-5-(2-cyano-4-trifluoromethylphenoxy)phenoxy]acetate,

n-Butyl 2-phenylthio-2-[2-nitro-5-(2-methyl-4-trifluoromethylphenoxy)phenoxy]acetate,

2-n-Butylthio-2-[2-nitro-5-(2-nitro-4-trifluoromethylphenoxy]acetonitrile,

2-Phenylthio-2-[2-nitro-5-(2-bromo-4-trifluoromethylphenoxy)phenoxy]acetamide,

2-Benzylthio-2-[2-nitro-5-(2-fluoro-4-trifluoromethylphenoxy)phenoxy]ethanol,

2-Phenoxy-2-[2-nitro-5-(2,6-dichloro-4-trifluoromethylphenoxy)phenoxy]-1-chloroethane,

2-Benzyloxy-2-[2-nitro-5-(2,4-bistrifluoromethylphenoxy)phenoxy]-N-dimethylacetamide,

2-Methoxy-2-[2-nitro-5-(2-chloro-4-trifluoromethylphenoxy)phenoxy]acetic acid,

Sodium 2-methoxy-2[2-nitro-5(2-chloro-4-trifluoromethylphenoxy)phenoxy]acetate,

Methyl 2-methoxy-2[2-nitro-5(2-chloro-4-trifluoromethylphenoxy)phenoxy]acetate,

1-Methoxy-1[2-nitro-5(2-chloro-4-trifluoromethylphenoxy]acetone,

Propargyl 2-methoxy-2[2-nitro-5(2-chloro-4-trifluoromethylphenoxy)phenoxy]acetate,

β,β,β,-Trifluoroethyl 2-methoxy-2[2-nitro-5-(2-chloro-4-trifluoromethylphenoxy)phenoxy]acetate,

β-Methoxyethyl 2-methoxy-2(2-nitro-5[2-chloro-4-trifluoromethylphenoxy)phenoxy]acetate,

β-Hydroxyethyl 2-methoxy-2-[2-nitro-5(2-chloro-4-trifluoromethylphenoxy)phenoxy]acetate,

Methoxyethoxyethyl 2-methoxy-2-[2-nitro-5(2-chloro-4-trifluoromethylphenoxy)phenoxy]acetate,

β-Chloroethyl 2-methoxy-2[2-nitro-5(2-chloro-4-trifluoromethylphenoxy)phenoxy]acetate,

Cyanomethyl 2-methoxy-2[2-nitro-5(2-chloro-4-trifluoromethylphenoxy)phenoxy]acetate,

β-Cyanoethyl 2-methoxy-2[2-nitro-5(2-chloro-4-trifluoromethylphenoxy)phenoxy]acetate,

β-Nitroethyl 2-methoxy-2[2-nitro-5(2-chloro-4-trifluoromethylphenoxy)phenoxy]acetate, and

β-Phenethyl 2-methoxy-2[2-nitro-5(2-chloro-4-trifluoromethylphenoxy)phenoxy]acetate,

The compounds of Formula I may be prepared by methods described in the literature for analogous compounds and, in particular, by methods such as shown by the examples.

Using the procedure described below, the diphenyl ether of Example 1 was evaluated for control of the following weeds among the following crops:

| Code | Common Name | Scientific Name |
|---|---|---|
| Weeds | | |
| BYG | barnyardgrass | *Echinochloa crusgalli* |
| BW | buckwheat | *Fagopyrum esculentum* |
| CKL | cocklebur | *Xanthium pensylvanicum* |
| DB | downybrome | *Bromus tectorum* |
| EUP | euphorbia | *Euphorbia heterophylla* |
| FOX | green foxtail | *Setaria viridis* |
| JOH | johnsongrass | *Sorghum halepense* |
| MA | marigold | *Tagetes* spp. |
| MG | morningglory | *Ipomoea* spp. |
| NUT | yellow nutsedge | *Cyperus esculentus* |
| SMT | smartweed | *Polygonum* spp. |
| TBW | tartary buckwheat | *Fagopyrum tataricum* |
| TOM | tomato | *Lycopersicon esculentum* |
| WO | wild oats | *Avena fatua* |
| VEL | velvetleaf | *Abutilon theophrasti* |

Crops

| | | |
|---|---|---|
| BAR | barley | *Hordeum sativum* |
| CN | maize | *Zea mays* |
| COT | cotton | *Gossypium hirsutum* |
| OT | oats | *Avena sativa* |
| RA | rape | *Brassica napus* |
| RI | rice | *Oryza sativa* |
| SB | sugarbeets | *Beta vulgaris* |
| SF | sunflower | *Helianthus annua* |
| SOY | soybeans | *Glycine max* |
| WHT | wheat | *Triticum aestivum* |

The following test procedure was employed. Seeds of selected crops and weeds are planted in soil in trays. For preemergence tests, the soil in the trays is sprayed with the test compound immediately after the planting. For postemergence tests, the seeds are allowed to germinate and after growing in the greenhouse for two weeks, the growing plants are treated with the test compound. The compound to be evaluated is dissolved in a suitable solvent, principally acetone, and sprayed over the flats using a carrier volume equivalent to 467 l/ha (50 US gallons per acre) at the rate of application specified in the Tables given below. About two weeks after application of the test compound, the state of growth of the plants is observed and the phytotoxic effect of each compound determined as follows: each species is evaluated on a scale of 0-100 in which 0 = no activity and 100 = total kill and the results for the monocots and dicots separately averaged.

The diphenyl ether of Example I was evaluated in the primary evaluation against weeds only, and in the secondary evaluation against weeds and eight major crops. The primary evaluation data is presented in Table II and the secondary evaluation data is presented in Table III.

The compound shows preemergence activity. It has excellent postemergence activity. Wheat, rice and corn are the most tolerant monocot crops, and soybeans may have acceptable tolerance.

The first postemergent test shown in Table IV was on four older and hardened-off weed species; cocklebur, marigold, tomato, and morningglory. The rice and wheat plants were younger and more tender than the weeds.

In the test shown in Table V the weeds were buckwheat, euphorbia, smartweed, and tartary buckwheat. Crops tested were barley, oats, rice, soybean, and wheat.

TABLE II

Primary Evaluation Data

| | % Weed Control | | | |
|---|---|---|---|---|
| | Preemergence | | Postemergence | |
| kg/ha | AM[1] | AD[2] | AM | AD |
| 2.24 | 81 | 96 | 100 | 100 |
| 0.56 | 48 | 58 | 100 | 100 |
| 0.13 | 8 | 0 | 84 | 100 |

Weed species were: Monocots -- BYG, DB, FOX, JON, NUT, WO;
Dicots -- CKL, MA, MG, TOM, VEL

[1] Average monocots
[2] Average dicots

TABLE III

Greenhouse Secondary Evaluation

% Weed Control or Crop Injury

| kg/ha | AM[1] | AD[2] | CN | COT | RA | RI | SB | SF | SOY | WHT |
|---|---|---|---|---|---|---|---|---|---|---|
| | Preemergence | | | | | | | | | |
| 2.24 | | | 90 | 25 | 100 | 0 | 100 | 30 | 20 | 40 |
| 1.12 | 44 | 58 | 80 | 0 | 80 | 0 | 100 | 5 | 0 | 0 |
| 0.56 | 25 | 28 | 25 | 10 | 90 | 0 | 99 | 0 | 0 | 0 |
| 0.28 | 22 | 26 | | | | | | | | |
| | Postemergence | | | | | | | | | |
| 2.24 | | | 85 | 100 | 99 | 100 | 100 | 75 | 50 | |
| 1.12 | 90 | 100 | 50 | 100 | 97 | 100 | 100 | 70 | 35 | |
| 0.56 | 86 | 100 | 30 | 50 | 80 | 100 | 100 | 60 | 25 | |
| 0.28 | 82 | 98 | | | | | | | | |

[1]Average monocot.
[2]Average dicot.

TABLE IV

Postemergence Evaluation on Older Plants[1]

% Weed Control or Crop Injury

| kg/ha | WHT | RI | CKL | MA | TOM | MG | AVE |
|---|---|---|---|---|---|---|---|
| 0.28 | 60 | 80 | 90 | 80 | 85 | 100 | 89 |
| 0.13 | 50 | 50 | 85 | 50 | 75 | 88 | 77 |
| 0.07 | 40 | 30 | 80 | 45 | 25 | 100 | 62 |
| 0.03 | 40 | 25 | 75 | 40 | 25 | 100 | 60 |
| 0.02 | 35 | 25 | 30 | 40 | 25 | 98 | 48 |
| 0.01 | - | 10 | 40 | 20 | 20 | 95 | 44 |
| | | | | | | | 63 |

[1] Weed plants were 8 weeks old. Rice and wheat were 2 weeks old.

TABLE V

Postemergence Evaluation on Hardened-off Weeds to Control Dicots

| | % Crop Injury or Weed Control | | | | | | | | |
|---|---|---|---|---|---|---|---|---|---|
| | Weeds | | | | Crops | | | | |
| kg/ha | BW | EUP | SMT | TBW | BAR | OT | RI | SOY | WHT |
| 0.56 | | | | | 78 | 90 | 55 | 85 | 40 |
| 0.28 | 95 | 95 | 100 | 100 | 68 | 88 | 40 | 78 | 40 |
| 0.13 | 90 | 96 | 100 | 100 | 80 | 75 | 33 | 65 | 30 |
| 0.07 | 80 | 83 | 95 | 100 | 70 | 60 | 5 | 58 | 35 |
| 0.03 | 68 | 45 | 90 | 100 | | | | | |
| AVE | 83 | 80 | 96 | 100 | | | | | |

The herbicidal activity of the diphenyl ethers of Examples 2-19 of the invention is exhibited on the following representative species:

| | | | Approx. # Seeds |
|---|---|---|---|
| Monocots | Barnyardgrass | (*Echinochloa crusgalli*) | 25 |
| | Downybrome | (*Bromus tectorum*) | 20 |
| | Foxtail | (*Setaria* spp) | 25 |
| | Johnsongrass | (*Sorghum halepense*) | 25 |
| | Nutsedge | (*Cyperus esculentus*) | 5 |
| | Wild Oat | (*Avena fatua*) | 20 |
| Dicots | Cocklebur | (*Xanthium pensylvanicum*) | 3 |
| | Marigold | (*Tagetes* spp) | 15 |
| | Morningglory | (*Ipomoea* spp) | 10 |
| | Tomato | (*Lycopersicon esculentum*) | 15 |
| | Velvetleaf | (*Abutilon theophrasti*) | 15 |

The test procedure described above is employed.

The results are given in Table VI.

TABLE VI

| Compound of Example No. | Rate 0.56 kg/ha Preemergent AM[1] | Rate 0.56 kg/ha Preemergent AD[2] | Rate 0.56 kg/ha Postemergent AM | Rate 0.56 kg/ha Postemergent AD | Rate 2.24 kg/ha Preemergent AM | Rate 2.24 kg/ha Preemergent AD | Rate 2.24 kg/ha Postemergent AM | Rate 2.24 kg/ha Postemergent AD |
|---|---|---|---|---|---|---|---|---|
| 1. | 25 | 68 | 92 | 100 | 78 | 100 | 98 | 100 |
| 2. | 18 | 79 | 83 | 100 | 67 | 99 | 93 | 100 |
| 3. | 38 | 82 | 88 | 100 | 69 | 94 | 98 | 100 |
| 4. | 43 | 94 | 88 | 100 | 90 | 94 | 97 | 100 |
| 5. | 51 | 100 | 82 | 100 | 73 | 100 | 89 | 100 |
| 6. | 12 | 74 | 50 | 99 | 58 | 90 | 64 | 100 |
| 7. | 0 | 31 | 10 | 46 | 3 | 95 | 17 | 80 |
| 8. | 0 | 0 | 7 | 36 | 2 | 52 | 13 | 64 |
| 9. | 22 | 78 | 20 | 90 | 63 | 96 | 58 | 98 |
| 10. | 58 | 76 | 20 | 100 | | | | |
| 11. | 28 | 60 | 22 | 88 | 83 | 70 | 28 | 96 |
| 12. | 40 | 81 | 61 | 98 | 79 | 96 | 68 | 99 |
| 13. | 0 | 22 | 3 | 8 | 3 | 61 | 7 | 10 |
| 14. | 18 | 60 | 10 | 70 | 61 | 75 | 10 | 83 |
| 15. | 47 | 64 | 27 | 61 | 74 | 78 | 45 | 93 |
| 16. | 37 | 52 | 28 | 65 | 75 | 86 | 40 | 71 |
| 17. | 0 | 38 | 7 | 14 | 0 | 32 | 8 | 56 |
| 18. | 40 | 79 | 61 | 100 | 73 | 80 | 70 | 100 |
| 19. | 2 | 59 | 20 | 84 | 48 | 100 | 17 | 82 |

[1] Average monocots

[2] Average dicots

The diphenyl ethers of Examples 1 - 19 were subjected to secondary greenhouse evaluations on the weeds and crops listed below. These evaluations were carried out using the procedure described above and the results are given in Table VII below. Except where noted by an asterisk, the results in Table VII were obtained at an application rate of 0.56 Kg/ha (0.5 lb/acre) of the diphenyl ether. As may be noted, the compounds are quite active, and in some cases selective herbicide-crop activity does not appear until quite low levels of herbicide are reached. In some cases, e.g. soybeans, the injury is only temporary.

WEEDS

MONOCOTS

| | | |
|---|---|---|
| ALG | ALEXANDER GRASS | Brachiaria plantaginea |
| BYG | BARNYARDGRASS | Echinochloa crus-galli |
| CAN | CANARY GRASS | Phalaris minor |
| DB | DOWNYBROME | Bromus techorum |
| FOX | FOXTAIL, GREEN | Setaria viridis |
| JON | JOHNSONGRASS | Sorghum halepense |
| NUT | NUTSEDGE, YELLOW | Cyperus esculentus |
| WO | WILD OAT | Avena fatua |

DICOTS

| | | |
|---|---|---|
| ANO | ANODA | Anoda cristata |
| BID | BIDENS | Bidens pilosa |
| BND | BINDWEED | Convolvulus arvensis |
| CD | CURLY DOCK | Rumex crispus |
| CKL | COCKLEBUR | Xanthium pensylvanicum |
| EUP | EUPHORBIA | Euphorbia heterophylla |
| MA | MARIGOLD | Tagetes spp. |
| MG | MORNINGGLORY | Ipomoea spp. |
| MT | MATRICARIA | Matricaria spp. |
| SIC | SICKLEPOD | Cassia obtusifolia |
| TBW | TARTARY BUCKWHEAT | Fagopyrum tataricum |
| TEA | TEAWEED | Sida spinosa |
| TOM | TOMATO | Lycopersicon esculentum |
| VEL | VELVETLEAF | Abutilon theophrasti |
| WC | WILD CARROT | Daucus carota |
| WKL | WHITE COCKLE | Lychnis alba |
| WM | WILD MUSTARD | Brassica kaber |
| WR | WILD RADISH | Raphanus raphanistrum |

CROPS

| | | |
|---|---|---|
| CN | CORN | *Zea mays* |
| COT | COTTON | *Gossypium hirsutum* |
| PNT | PEANUTS | *Arachis hypogaea* |
| RA | RAPE | *Brassica napus* |
| RI | RICE | *Oryza sativa* |
| SB | SUGARBEET | *Beta vulgaris* |
| SF | SUNFLOWER | *Helianthus annuus* |
| SOY | SOYBEAN | *Glycine max* |
| WHT | WHEAT | *Triticum spp.* |

TABLE VII

| Ex. | ALG | BYG | CAN | DB | FOX | JON | NUT | WO | ANO | BID | BND | CD | CKL |
|---|---|---|---|---|---|---|---|---|---|---|---|---|---|
| 1. | 10 | 100 | 35 | 99 | 100 | 80 | 40 | 99 | 90 | 100* | 90 | 100* | 100 |
| 2. | | 100 | | 95 | 100 | 50 | 40 | 80 | | | 100* | 100* | 100 |
| 3. | | 99 | | 99 | 100 | 40 | 10 | 90 | 100* | 100* | 100* | 100* | 100 |
| 4. | | 100 | | 99 | 100 | 50 | 30 | 100 | | | | | 100 |
| 5. | | 100 | | 40 | 100 | 40 | 20 | 80 | | | 100* | 100* | 100 |
| 6. | | 97 | | 40 | 40 | 15 | 10 | 25 | | | 99* | 100* | 100 |
| 7. | 0 | 10 | 20 | 0 | 25 | 10 | 0 | 10 | 15 | | 85* | 80* | |
| 8. | 0 | 10 | 0 | 0 | 10 | 10 | 0 | 5 | 10 | | 30* | 95* | 10 |
| 9. | | 40 | | 10 | 30 | 15 | 10 | 10 | | | | | 100 |
| 10. | | | | | | | | | | | | | |
| 11. | | | | | | | | | | | | | |
| 12. | 70* | 30* | 75* | 40* | 90* | 20* | 15* | 25* | 60* | | 100* | 100* | 75* |
| 13. | 0 | 0 | 0 | 0 | 0 | 0 | 0 | 5 | 5 | | 20* | 95* | 10 |
| 14. | 0 | 0 | 0 | 0 | 25 | 0 | 10 | 5 | 50 | | 85* | 100* | 15 |
| 15. | 0 | 15 | 30 | 0 | 70 | 40 | 10 | 20 | 15 | | 95 | | 50 |
| 16. | 10 | 10 | 35 | 0 | 40 | 20 | 0 | 25 | 40 | | 80* | 100* | 15 |
| 17. | 0 | 0 | 10 | 0 | 50 | 20 | 0 | 5 | 40 | | 50* | 100* | 10 |
| 18. | 20* | 0* | 40* | 30* | 25* | 0 | 10* | 10* | 100* | 100* | 20* | 100* | 100* |
| 19. | 25* | 20* | 15* | 10* | 25* | 10* | 0* | 15* | 99* | 100* | 75* | 99* | 80* |

TABLE VII (CONT'D)

| Ex. | EUP | MA | MG | MT | SIC | TBW | TEA | TOM | VEL | WC | WKL | WM | WR |
|---|---|---|---|---|---|---|---|---|---|---|---|---|---|
| 1. | 100 | 100 | 100 | 100* | 20 | 100* | 90 | 100 | 100 | 95* | 100* | 98 | 100 |
| 2. | | 100 | 100 | | | 100* | | 100 | 100 | | | 99* | 100* |
| 3. | 100* | 100 | 100 | | 85* | 100* | 98* | 100 | 100 | 100* | 100* | 100* | 100 |
| 4. | | 100 | 100 | | | | | 100 | 100 | | | | |
| 5. | | 100 | 100 | | | 100* | | 97 | 100 | | | 100* | 100* |
| 6. | | 100 | 100 | | | 100* | | 100 | 100 | 85* | 100* | 93* | 100* |
| 7. | 10 | 99 | 30 | 95* | 5 | 65* | 40 | 0 | 50 | 0 | 100* | 30 | 85 |
| 8. | 25 | 75 | 20 | 95* | 5 | 100* | 10 | 20 | 60 | 20* | 100* | 20 | 15 |
| 9. | | 100 | 100 | | | | | 97 | 100 | | | | |
| 10. | | | | | | | | | | | | | |
| 11. | | | | | | | | | | | | | |
| 12. | 98* | 100* | 98* | 98* | 10* | 100* | 100* | 100* | 100* | 30* | 100* | 100* | 100* |
| 13. | 10 | 0 | 10 | 0* | 5 | 10* | 20 | 0 | 15 | 20* | 100* | 20 | 15 |
| 14. | 20 | 80 | 40 | 98* | 5 | 100* | 20 | 10 | 100 | 0* | 100* | 80 | 90 |
| 15. | 100 | 100 | 70 | | 10 | | 70 | 30 | 100 | | | 98 | 100 |
| 16. | 25 | 90 | 75 | 98* | 5 | 100* | 50 | 20 | 100 | 0* | 100* | 50 | 35 |
| 17. | 20 | 10 | 20 | 30* | 0 | 90* | 25 | 10 | 100 | 0* | 25 | 20 | |
| 18. | 90* | 100* | 99* | | 90* | 99* | 100* | 85* | 100* | 85* | 100 | 70* | 50* |
| 19. | 99* | 100* | 80* | 100* | 60 | 100* | 95 | 99* | 100* | 20* | 100* | 95* | 97* |

TABLE VII (CONT'D)

| Ex. | CN | COT | PNT | RA | RI | SB | SF | SOY | WHT |
|---|---|---|---|---|---|---|---|---|---|
| 1. | 30 | 100 | 25 | 50 | 80 | 100 | 100 | 60 | 25 |
| 2. | 0 | 100 | 25 | 100 | 90 | 95 | 100 | 98 | 50 |
| 3. | 30 | 100 | 35 | 100 | 95 | 99 | 100 | 99 | 30 |
| 4. | 10 | 100 | 30 | 100 | 90 | 99 | 100 | 95 | 30 |
| 5. | 15 | 100 | 30 | 100 | 50 | 98 | 100 | 90 | 25 |
| 6. | 5 | 100 | 35 | 100 | 70 | 30 | 100 | 75 | 15 |
| 7. | | 25 | | 10 | 15 | 5 | 40 | 10 | 0 |
| 8. | 0 | 15 | | 0 | 50 | 5 | 20 | 20 | 0 |
| 9. | 5 | 100 | 25 | 98 | 70 | 80 | 95 | 70 | 10 |
| 10. | | | | | | | | | |
| 11. | | | | | | | | | |
| 12. | 5 | 80 | | 95 | 25 | 80 | 85 | 15 | 25 |
| 13. | 0 | 10 | | 0 | 0 | 5 | 5 | 10 | 0 |
| 14. | 5 | 20 | | 20 | 15 | 10 | 15 | 15 | 5 |
| 15. | 0 | 75 | | 100 | 25 | 75 | 80 | 15 | 0 |
| 16. | 0 | 15 | | 0 | 5 | 5 | 20 | 15 | 0 |
| 17. | 0 | 20 | | 20 | 5 | 0 | 10 | 15 | 0 |
| 18. | 5 | 100 | | 100 | 50 | 90 | 100 | 15 | 15 |
| 19. | 10 | 35 | | 40 | 40 | 20 | 95 | 20 | 30 |

* Data are for 0.28 kg/ha (0.25 lb/acre)

There should also be mentioned as specific compounds within the scope of Formula I all the individual compounds of Formula I hereinbefore disclosed but in which (referring to the symbols used in Formula I):

(a) X in the individual ether in question is replaced by a different group or atom selected from each and every one of H, Br, Cl, F, I and $CF_3$;

(b) Y in the individual ether in question is replaced by a different group or atom selected from each and every one of H, Br, Cl, F, I and $CF_3$;

(c) W in the individual ether in question is replaced by a different group or atom selected from each and every one of O, S, SO and $SO_2$;

(d) R in the individual ether in question is replaced by a different group selected from each and every one of <u>n</u>-$C_4H_9$, phenyl, 4-chlorophenyl, 3,4-dichlorophenyl, 4-fluorophenyl, 4-bromophenyl, 3-iodophenyl, 4-trifluoromethylphenyl, benzyl, $C_6H_5(CH_2)_4$- and phenethyl;

(e) Q in the individual ether in question is different and is selected from each and every one of $(CH_2)_0$, $CH_2$, $(CH_2)_2$, $(CH_2)_3$, $(CH_2)_4$, CH=CH, $CH_2CH{=}CH_2$ and $CH_2CH_2CH{=}CHCH_2CH_2$;

(f) Z in the individual ether in question is replaced by a different group selected from each and every one of $CO_2H$, $CO_2^-$, $CONH_2$, CN, $CH_2OH$, $CH_2Cl$ and $CH_2OCH_3$; and

(g) Z in the individual ether in question is replaced by a different group selected from each and every one of $CO_2R^1$, $COSR^1$, $CONHR^1$, $CON(R^1)_2$, $COR^1$, $CO_2(CR^2R^3)_mCO_2R^1$ and $OR^1$ wherein $R^1$ in each of the last seven groups is selected from each and every one of $CH_3$, <u>n</u>-$C_4H_9$, $CH_2CH{=}CH_2$, $CH_2CH_2CH{=}CHCH_2CH_3$, $CH_2CN$, $(CH_2)_4CN$, $C{\equiv}CCH_3$, $C{\equiv}CCH_2CH_2CH_2CH_3$ $CH_2CH_2Cl$, $CH_2CH_2CH_2CH_2Cl$, $CH_2CHBrCH_3$, $CH_2OH$, $(CH_2)_3CH_2OH$, $CH_2OCH_2CH_2CH_2CH_3$, $(CH_2)_4OCH_3$, $CH_2O(CH_2)_4OCH_3$, $(CH_2)_4OCH_2O(CH_2)_3CH_3$, $CH_2CF_3$, $(CH_2)_2NO_2$, $(CH_2)_4NO_2$, cyclopropyl, cycloactyl, phenylmethyl and phenyl-<u>n</u>-butyl; and wherein when Z is $CO_2(CR^2R^3)_mCO_2R^1$, the group

$-(CR^2R^3)_m-$ is selected from each and every one of $CH_2$, $CH(CH_3)$, $C(CH_3)_2$, $CH_2CH_2$, $CH_2CH(CH_3)$, $CH(CH_3)CH(CH_3)$, $(CH_2)_3$, $(CH_2)_2CH(CH_3)$ and $(CH_2)_2C(CH_3)_2$.

Although in the foregoing description general directions have been given for preparing herbicidal compositions containing one or more of the diphenyl ethers of Formula I, examples of specific herbicidal compositions are given below. In these compositions the abbreviation "AI" indicates active ingredient which is the diphenyl ether of Example 1, 3, 4 or 5 above or, depending on solubility etc properties, a diphenyl ether of any one of the other foregoing examples. As will be understood the compositions set forth below may, depending on the AI content, require dilution with additional liquid (usually water) or solid agronomically acceptable diluent or carrier.

Solid Ready-For-Use Preparations

Dust

| | % by weight |
|---|---|
| AI | 0.1 |
| Talc | 99.9 |

Granules (0.5-1.5 mm average diameter)

| | % by weight |
|---|---|
| AI | 0.1 |
| Attapulgite clay (20.40 mesh U.S. Standard Screen Series) | 99.9 |

Liquid Preparations

Solution

| | % by weight |
|---|---|
| AI | 0.1 |
| Acetone/water | 99.9 |

Liquid Preparations (continued)

Emulsion

| | % by weight |
|---|---|
| Emulsifiable concentrate containing 50 wt% AI, 5 wt% calcium dodecylbenzene sulfonate, 5 wt% octylphenoxypoly(ethyleneoxy)ethanol containing an average of 9 ethyleneoxy groups (surfactant) | 0.1 |
| Water | 99.9 |

Suspension

| | |
|---|---|
| Wettable powder containing 50 wt% AI, 21.5 wt% kaolin, 25.0 wt% precipitated silica, 3.0 wt% sodium nonylnaphthalenesulfonate, sodium lauryl sulfate | 0.1 |
| Xanthan gum (long chain polysaccharide obtained by the fermentation of molasses) | 0.25 |
| Propylene glycol | 5.0 |
| Water | 94.65 |

Solid Concentrate

Dust Concentrate

| | % by weight |
|---|---|
| AI | 95.0 |
| Talc | 5.0 |

Liquid or Semi-Liquid Water-Soluble or Water-Dispersible Concentrates

Solution

| | % by weight |
|---|---|
| AI | 1.0 |
| Octylphenoxypoly(ethyleneoxy) ethanol containing an average of 9 ethyleneoxy groups (surfactant) | 1.5 |
| Dimethylformamide | 97.5 |

Flowable Liquid Suspension

| | |
|---|---|
| AI | 50.0 |
| Octylphenoxypoly(ethyleneoxy) ethanol containing an average of 9 ethyleneoxy groups (surfactant) | 2.0 |
| Sodium polyacrylate | 0.5 |
| Xanthan gum | 0.05 |
| Propylene glycol | 5.0 |
| Water | 42.45 |

Paste

| | |
|---|---|
| AI | 5.0 |
| Palm oil | 28.0 |
| Propylene glycol | 5.0 |
| Xylene | 21.0 |
| Octylphenoxypoly(ethyleneoxy) ethanol containing an average of 9 ethyleneoxy groups (surfactant) | 10.0 |
| Calcium stearate | 4.0 |

Claims:

1. A compound of the formula:

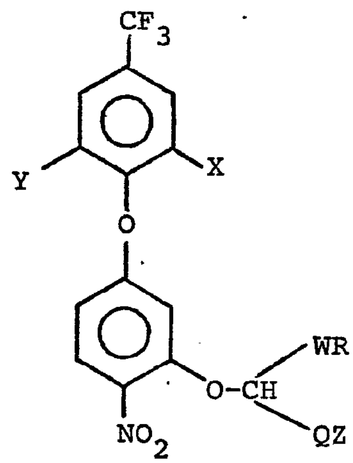

Formula I

wherein X is hydrogen, halo, trifluoromethyl, $(C_1-C_4)$alkyl, cyano, or nitro;

Y is hydrogen, halo, or trifluoromethyl;

W is O or $S(O)_p$, wherein p is 0 to 2;

R is $(C_1-C_4)$alkyl, phenyl, halo-substituted phenyl, trifluoromethyl-substituted phenyl, or phenyl-$(C_1-C_4)$alkyl;

Q is $(CH_2)_n$ wherein n is 0 to 4, or $(C_2-C_6)$-alkenylene;

Z is $CO_2R^1$, $CO_2H$, $CO_2^-$, $CONH_2$, $CONHR^1$, $COSR^1$,

$CON(R^1)_2$, CN, $CH_2OH$, $CH_2Cl$, $COR^1$, $CH_2OCH_3$,

$CO_2(CR^2R^3)_mCO_2R^1$ or $OR^1$

wherein

$R^1$ is $(C_1-C_4)$alkyl, $(C_3-C_6)$alkenyl, cyano$(C_1-C_4)$-alkyl, $(C_3-C_6)$alkynyl, $(C_2-C_4)$haloalkyl, $(C_1-C_4)$hydroxyalkyl, $(C_1-C_4)$alkoxy$(C_1-C_4)$alkyl, $(C_1-C_4)$alkoxy$(C_1-C_4)$alkoxy$(C_1-C_4)$alkyl trifluoroethyl, $(C_2-C_4)$nitroalkyl, $(C_3-C_8)$-cycloalkyl or phenyl$(C_1-C_4)$alkyl,

$R^2$ and $R^3$ are the same or different and are selected from hydrogen and methyl, the $-CR^2R^3-$ groups being the same or different when m is >1, and, when Z is $CO_2H$, an agronomically acceptable salt of a compound of Formula I.

2. A compound or salt as claimed in Claim 1, wherein the compound of Formula I in Claim 1 has the structure:

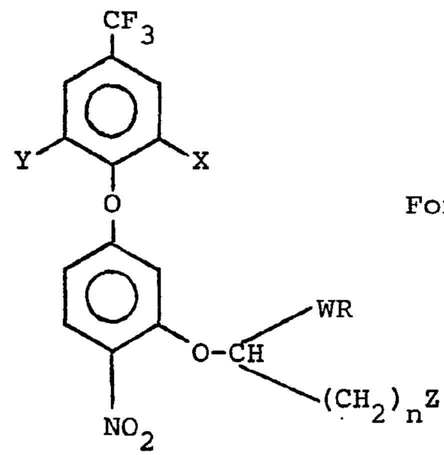

Formula II

wherein X is hydrogen, halo, trifluoromethyl, $(C_1-C_4)$-alkyl, cyano, or nitro;

Y is hydrogen, halo, or trifluoromethyl;

W is O or $SO_p$;

R is $(C_1-C_4)$alkyl, phenyl or phenyl$(C_1-C_4)$alkyl;

n is from 0 to 4;

p is from 0 to 2; and

Z is $CO_2R^1$, $CO_2H$, $CO_2^-$, $CONH_2$, $CONHR^1$, COSR, $CON(R^1)_2$, CN, $CH_2OH$, $CH_2Cl$, or $COR^1$, wherein $R^1$ is $(C_1-C_4)$alkyl or $(C_2-C_4)$alkenyl.

3. A compound, or salt, as claimed in Claim 2, wherein WR is alkoxy, alkylthio, phenylthio, benzylthio, phenoxy or benzyloxy and Z is carboxy or alkoxycarbonyl.

4. A compound, or salt, as claimed in any one of

Claims 1 to 3, wherein Y is hydrogen and X is chlorine or cyano.

5. A compound, or salt, as claimed in any one of Claims 1 to 4, wherein R is $(C_1-C_4)$alkyl, phenyl or phenyl$(C_1-C_4)$alkyl and Z is $CO_2R^1$, $CO_2H$, $CO_2^-$, $CONH_2$, $CONHR^1$, $CON(R^1)_2$, CN, $CH_2OH$, $CH_2Cl$ or $COCH_3$, $R^1$ being $(C_1-C_4)$alkyl.

6. A compound as claimed in Claim 1, wherein the compound of Formula I in Claim 1 has the structure:

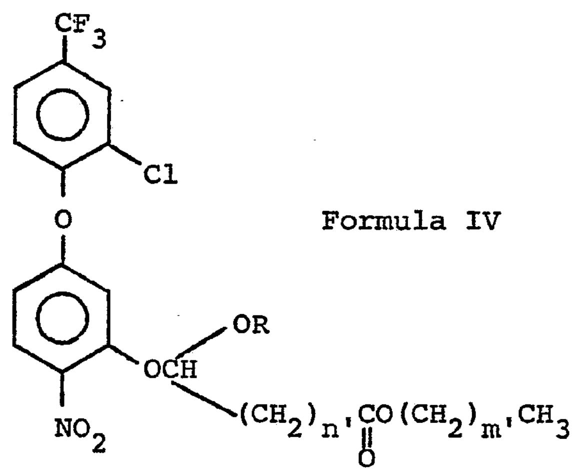

Formula IV

wherein R is $(C_1-C_4)$alkyl;
m' is 0 or 1; and
n' is 0 or 1.

7. A compound as claimed in Claim 1, wherein X is Cl, Y is H, WR is $OCH_3$, and QZ is $CO_2R^1$ wherein $R^1$ is $(C_1-C_4)$alkyl.

8. A compound as claimed in Claim 7, wherein $R^1$ is methyl.

9. A compound as claimed in Claim 1, wherein X is Cl, Y is H, WR is $OCH_3$, and QZ is $CO_2R^1$ wherein $R^1$ is $(C_3-C_6)$alkenyl.

10. A compound as claimed in Claim 9, wherein $R^1$ is allyl.

11. A compound or salt as claimed in Claim 2, wherein Y is hydrogen and WR, X, $(CH_2)_nZ$ and $(CH_2)_nZ$ salt are one of the following:

| WR | X | $(CH_2)_nZ$ | $(CH_2)_nZ$ salt |
|---|---|---|---|
| $OCH_3$ | Cl | | $CO_2Na$ |
| $OC_2H_5$ | Cl | $CO_2CH_3$ | |
| $SCH_3$ | Cl | $CO_2C_2H_5$ | |
| $SOCH_3$ | Cl | $CO_2C_2H_5$ | |
| $OCH_3$ | Cl | $CO_2C_2H_5$ | |
| $OCH_3$ | Cl | $C_2H_4OH$ | |
| $OCH_3$ | Cl | $COCH_3$ | |
| $SO_2CH_3$ | Cl | $CO_2C_2H_5$ | |
| $OC_6H_5$ | Cl | $CO_2CH_3$ | |
| $OCH_2C_6H_5$ | Cl | $CO_2CH_3$ | |
| $OCH_3$ | Cl | $CO_2H$ | |
| $OCH_3$ | Cl | $CON(CH_3)_2$ | |
| $OCH_3$ | Cl | $CH_2CO_2CH_3$ | |
| $OCH_3$ | CN | $CH_2CO_2CH_3$ | |
| $OCH_3$ | CN | $CO_2CH_3$ or | |
| $OCH_3$ | Cl | CN | |

12. A compound as claimed in Claim 2, wherein X is chlorine, Y is hydrogen, WR is $OCH_3$ and $(CH_2)_nZ$ is $CO_2CH(CH_3)_2$ or $CO_2CH_2CH{=}CH_2$.

13. A compound as claimed in Claim 1, wherein X is chlorine, Y is hydrogen, W is O and (a) R is $C_2H_5$ and QZ is $CO_2C_2H_5$, (b) R is $CH_2C_6H_5$ and QZ is $CO_2CH_2C_6H_5$, (c) R is $C_6H_5$ and QZ is CN or (d) R is $CH_3$ and QZ is $CH_2OCH_3$, $(CH_2)_2CO_2CH_3$, $CO_2CH_2CO_2CH_3$ or $CO_2CH(CH_3)CO_2CH_3$.

14. A herbicidal composition containing, as active ingredient, at least one compound or salt as defined in any one of Claims 1 to 13 and an agronomically acceptable carrier or diluent for the active ingredient.

15. A composition as claimed in Claim 14 containing 0.1 to 95% by weight of said active ingredient.

16. A composition as claimed in Claim 14 or 15 in the form of (a) a dust or granules or (b) a formulation which contains a surfactant and which is a wettable powder, concentrated liquid suspension, solution, water-dispersible paste, emulsifiable concentrate or flowable emulsion concentrate.

17. A method of combating weeds which comprises applying to growing weeds or to a growth medium prior to emergence of weeds therefrom a compound or salt as defined in any one of Claims 1 to 13 in an amount sufficient to combat the growth of the weeds.

18. A method as claimed in Claim 17 as applied to the combating of weeds in an agronomic crop area.

19. A method as claimed in Claim 18 wherein the agronomic crop is a cereal crop.

Claims:

1. A herbicidal composition containing an active herbicidal component and an agronomically acceptable diluent or carrier therefor, characterized in that the active component comprises one or more diphenyl ethers of the formula:

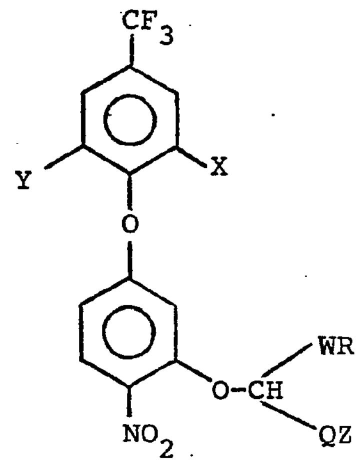

Formula I

wherein X is hydrogen, halo, trifluoromethyl, $(C_1-C_4)$alkyl, cyano, or nitro;

Y is hydrogen, halo, or trifluoromethyl;

W is O or $S(O)_p$, wherein p is 0 to 2;

R is $(C_1-C_4)$alkyl, phenyl, halo-substituted phenyl, trifluoromethyl-substituted phenyl, or phenyl-$(C_1-C_4)$alkyl;

Q is $(CH_2)_n$ wherein n is 0 to 4, or $(C_2-C_6)$-alkenylene;

Z is $CO_2R^1$, $CO_2H$, $CO_2^-$, $CONH_2$, $CONHR^1$, $COSR^1$,

$CON(R^1)_2$, CN, $CH_2OH$, $CH_2Cl$, $COR^1$, $CH_2OCH_3$,

$CO_2(CR^2R^3)_mCO_2R^1$ or $OR^1$

wherein

$R^1$ is $(C_1-C_4)$alkyl, $(C_3-C_6)$alkenyl, cyano$(C_1-C_4)$-alkyl, $(C_3-C_6)$alkynyl, $(C_2-C_4)$haloalkyl, $(C_1-C_4)$hydroxyalkyl, $(C_1-C_4)$alkoxy$(C_1-C_4)$alkyl, $(C_1-C_4)$alkoxy$(C_1-C_4)$alkoxy$(C_1-C_4)$alkyl trifluoroethyl, $(C_2-C_4)$nitroalkyl, $(C_3-C_8)$-cycloalkyl or phenyl$(C_1-C_4)$alkyl,

$R^2$ and $R^3$ are the same or different and are selected from hydrogen and methyl, the $-CR^2R^3-$ groups being the same or different when m is >1, and, when Z is $CO_2H$, an agronomically acceptable salt of a compound of Formula I.

2. A composition as claimed in Claim 1, characterized in that it contains a compound of Formula II below or a salt of a compound of Formula II

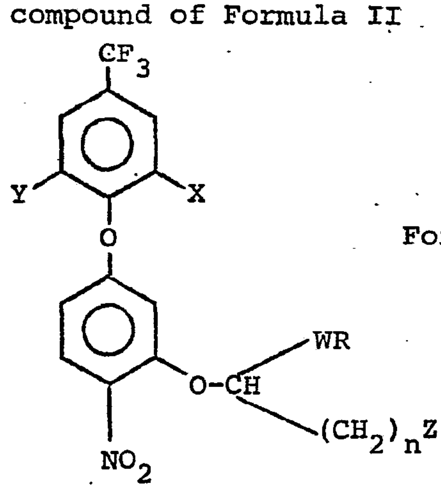

Formula II

wherein X is hydrogen, halo, trifluoromethyl, $(C_1-C_4)$-alkyl, cyano, or nitro;
Y is hydrogen, halo, or trifluoromethyl;
W is O or $SO_p$;
R is $(C_1-C_4)$alkyl, phenyl or phenyl$(C_1-C_4)$alkyl;
n is from 0 to 4;
p is from 0 to 2; and
Z is $CO_2R^1$, $CO_2H$, $CO_2^-$, $CONH_2$, $CONHR^1$, COSR, $CON(R^1)_2$, CN, $CH_2OH$, $CH_2Cl$, or $COR^1$, wherein $R^1$ is $(C_1-C_4)$alkyl or $(C_2-C_4)$alkenyl.

3. A compound, or salt, as claimed in Claim 2, wherein WR is alkoxy, alkylthio, phenylthio, benzylthio, phenoxy or benzyloxy and Z is carboxy or alkoxycarbonyl.

4. A compound, or salt, as claimed in any one of

Claims 1 to 3, wherein Y is hydrogen and X is chlorine or cyano.

5. A composition, or salt, as claimed in any one of Claims 1 to 4, wherein R is $(C_1-C_4)$alkyl, phenyl or phenyl$(C_1-C_4)$alkyl and Z is $CO_2R^1$, $CO_2H$, $CO_2^-$, $CONH_2$, $CONHR^1$, $CON(R^1)_2$, CN, $CH_2OH$, $CH_2Cl$ or $COCH_3$, $R^1$ being $(C_1-C_4)$alkyl.

6. A composition as claimed in Claim 1, wherein the compound of Formula I in Claim 1 has the structure:

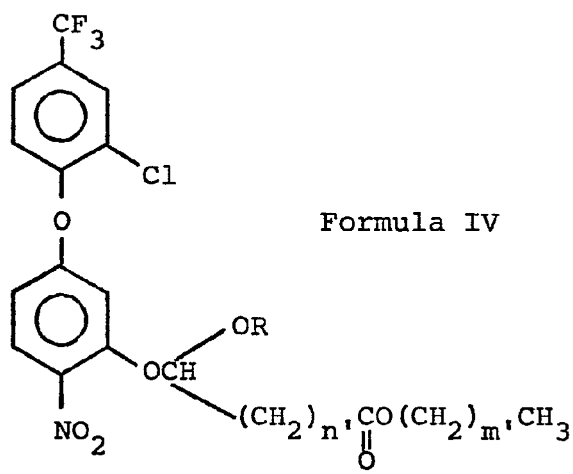

Formula IV

wherein R is $(C_1-C_4)$alkyl;
m' is 0 or 1; and
n' is 0 or 1.

7. A composition as claimed in Claim 1, wherein X is Cl, Y is H, WR is $OCH_3$, and QZ is $CO_2R^1$ wherein $R^1$ is $(C_1-C_4)$alkyl.

8. A composition as claimed in Claim 7, wherein $R^1$ is methyl.

9. A composition as claimed in Claim 1, wherein X is Cl, Y is H, WR is $OCH_3$, and QZ is $CO_2R^1$ wherein $R^1$ is $(C_3-C_6)$alkenyl.

10. A composition as claimed in Claim 9, wherein $R^1$ is allyl.

11. A composition or salt as claimed in Claim 2, wherein Y is hydrogen and WR, X, $(CH_2)_nZ$ and $(CH_2)_nZ$ salt are one of the following:

| WR | X | $(CH_2)_nZ$ | $(CH_2)_nZ$ salt |
|---|---|---|---|
| $OCH_3$ | Cl | | $CO_2Na$ |
| $OC_2H_5$ | Cl | $CO_2CH_3$ | |
| $SCH_3$ | Cl | $CO_2C_2H_5$ | |
| $SOCH_3$ | Cl | $CO_2C_2H_5$ | |
| $OCH_3$ | Cl | $CO_2C_2H_5$ | |
| $OCH_3$ | Cl | $C_2H_4OH$ | |
| $OCH_3$ | Cl | $COCH_3$ | |
| $SO_2CH_3$ | Cl | $CO_2C_2H_5$ | |
| $OC_6H_5$ | Cl | $CO_2CH_3$ | |
| $OCH_2C_6H_5$ | Cl | $CO_2CH_3$ | |
| $OCH_3$ | Cl | $CO_2H$ | |
| $OCH_3$ | Cl | $CON(CH_3)_2$ | |
| $OCH_3$ | Cl | $CH_2CO_2CH_3$ | |
| $OCH_3$ | CN | $CH_2CO_2CH_3$ | |
| $OCH_3$ | CN | $CO_2CH_3$ or | |
| $OCH_3$ | Cl | CN | |

12. A composition as claimed in Claim 2, wherein X is chlorine, Y is hydrogen, WR is $OCH_3$ and $(CH_2)_nZ$ is $CO_2CH(CH_3)_2$ or $CO_2CH_2CH{=}CH_2$.

13. A composition as claimed in Claim 1, wherein X is chlorine, Y is hydrogen, W is O and (a) R is $C_2H_5$ and QZ is $CO_2C_2H_5$, (b) R is $CH_2C_6H_5$ and QZ is $CO_2CH_2C_6H_5$, (c) R is $C_6H_5$ and QZ is CN or (d) R is $CH_3$ and QZ is $CH_2OCH_3$, $(CH_2)_2CO_2CH_3$, $CO_2CH_2CO_2CH_3$ or $CO_2CH(CH_3)CO_2CH_3$.

14. A method of combating weeds which comprises applying to growing weeds or to a growth medium prior to emergence of weeds therefrom a composition as claimed in any one of Claims 1 to 13 in an amount sufficient to combat the growth of the weeds.

15. A method as claimed in Claim 14 as applied to the combating of weeds in an agronomic crop area.

16. A method as claimed in Claim 15 wherein the agronomic crop area is a cereal crop.

European Patent Office

# EUROPEAN SEARCH REPORT

0022626

Application number EP 80302049.4

| Category | Citation of document with indication, where appropriate, of relevant passages | Relevant to claim |
|---|---|---|
| | EP - A1 - 0 001 641 (CIBA-GEIGY AG) (02-05-1979)<br>+ Pages 12-33 +<br>-- | 1,14, 17,AT1, 14 |
| | DE - A1 - 2 632 581 (HOECHST AG)<br>+ Claims; pages 14-23 +<br>---- | 1,14, 17,AT1, 14 |

**CLASSIFICATION OF THE APPLICATION (Int. Cl. 3)**

C 07 C 79/35
C 07 C 103/34
C 07 C 121/38
C 07 C 147/02
C 07 C 147/14
C 07 C 149/20
A 01 N 39/02

**TECHNICAL FIELDS SEARCHED (Int.Cl. 3)**

C 07 C 79/00
C 07 C 103/00
C 07 C 121/00
C 07 C 147/00
C 07 C 149/00
A 01 N 39/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 02-09-1980 | HOFBAUER |